Europäisches Patentamt

European Patent Office

Office européen des brevets

(11) Numéro de publication: **0 526 318 B1**

# FASCICULE DE BREVET EUROPEEN

(45) Date de publication de fascicule du brevet: **14.06.95**    (51) Int. Cl.⁶: **C07C 209/16**

(21) Numéro de dépôt: **92402154.6**

(22) Date de dépôt: **24.07.92**

(54) **Procédé pour la préparation d'alkylamines secondaires aliphatiques dissymétriques.**

(30) Priorité: **29.07.91 FR 9109615**

(43) Date de publication de la demande:
**03.02.93 Bulletin 93/05**

(45) Mention de la délivrance du brevet:
**14.06.95 Bulletin 95/24**

(84) Etats contractants désignés:
**AT BE CH DE DK ES FR GB GR IT LI LU NL PT SE**

(56) Documents cités:
**GB-A- 1 585 480**
**US-A- 3 223 734**

(73) Titulaire: **CECA S.A.**
**4 - 8, Cours Michelet**
**La Défense 10**
**F-92800 Puteaux (FR)**

(72) Inventeur: **Forquy, Christian**
**Ouartier Coos**
**F-64360 Monein (FR)**

(74) Mandataire: **Haicour, Philippe**
**Elf Atochem S.A., D.P.I.**
**La Défense 10 - Cédex 42**
**F-92091 Paris La Defense (FR)**

**Description**

La présente invention est relative à un procédé d'obtention industrielle d'amines secondaires dissymétriques, de formule générale R-NH-R′, où R est une chaîne grasse et R′ un reste aliphatique court, plus particulièrement un méthyle.

Alors qu'on dispose de procédés industriels pour préparer sélectivement des amines primaires $RNH_2$ ou des amines tertiaires diméthylées $RN(CH_3)_2$, on ne connaît pas de procédé sélectif pour préparer des amines secondaires monométhylées. Par sélectif, on entend précisément un procédé qui produit l'amine secondaire monométhylée avec un minimum d'amine primaire et d'amine tertiaire diméthylée correspondantes.

Des spécifications pour les dérivés envisagés doivent évidemment tenir compte des utilisations auxquelles on les destine. On compte parmi celles-ci l'obtention de produits intermédiaires pour la détergence qui doivent posséder des structures à la fois oxyalkylées et quaternisables à une seule chaîne aliphatique longue. Si on peut espérer séparer par distillation les amines à une seule chaîne grasse des amines à deux ou trois chaînes grasses, on ne pourra pas séparer convenablement les amines $RNH_2$ et $RN(CH_3)_2$ des amines secondaires $RNHCH_3$. En effet, les points d'ébullition pour une même longueur de chaîne alkyle sont très proches (approx.10 °C), et dès lors que l'on part de matières premières qui sont des coupes avec des longueurs de chaîne alkyle telles que C12/C14, C14/C16, C16/C18 ou C16/C22, ce qui est le cas général dans cette chimie, toute séparation des amines $RNH_2$, $RN(CH_3)_2$ et $RNHCH_3$ devient illusoire. On peut donc tolérer un certain pourcentage d'amines dialkyles et trialkyles, parce que séparables par distillation, dans des proportions qui, toutefois, n'obèrent pas l'économie du procédé. On peut aussi admettre un taux raisonnable de diméthylamines, qui suivront sans conséquence grave, les dérivés des méthylalkylamines, comme dérivés quaternisés. La contrainte pèse donc surtout sur la présence des monoalkylamines.

On vise ainsi les spécifications d'amines secondaires dissymétriques commerciales suivantes :

| | |
|---|---|
| $RNHCH_3$ | min. 85 % |
| $RNH_2$ | max. 8 % |
| $RN(CH_3)_2$ | max. 2 % |
| $R_2NH + R_2NCH_3$ | max. 6 % |
| $R_3N$ | max. 1 % |
| Non amine | max. 2 % |

pour le respect desquelles il est nécessaire de disposer d'un procédé sélectif en amine secondaire $RNHCH_3$.

On trouve décrits dans l'art antérieur plusieurs réactions et procédés pour obtenir les amines secondaires monométhylées, notamment :

- le brevet français n°1 595 771, (HENKEL), qui décrit la mise en oeuvre de la réaction entre un acide gras et la monométhylamine en présence d'un catalyseur zinc-aluminium à 310 °C, qui cependant n'est pas suffisamment sélective en amine secondaire monométhylée puisqu'on forme au moins 10 % d'amine tertiaire diméthylée ;
- le brevet européen n° EP 34 480, (R. E. GRIGG), dans lequel l'utilisation de complexes d'hydrures de métaux nobles du type $RhH(PPh_3)_4$ permet la monométhylation sélective, par exemple par le méthanol, d'amines telles que la butylamine, la pyrrolidine ou la cyclohexylamine; sans que toutefois ces catalyseurs soient adaptés à une production industrielle d'amines grasses monométhylées ;
- les brevets français n° 2 370 029, 2 370 030, 2 370 031 et 2 405 921, les brevets US n° 4 206 149-4 206 150, qui décrivent la mise en oeuvre de la réaction entre un alcool gras et la monométhylamine sur des catalyseurs à base de cuivre, supportés sur alumine et contenant au moins l'un des éléments suivants, Cr, Zn, W, Re, Sn ; brevets français dans lesquels les sélectivités en amines primaire et tertiaire diméthylées ne sont pas indiquées, mais où il est indiqué qu'il se forme généralement des quantités importantes (>15 % poids) d'amines dialkylées ; brevets américains relatant des réactions catalysées sur $Cu-Re/Al_2O_3$ et $Cu-W/Al_2O_3$, où l'on voit que la teneur en $RN(CH_3)_2$ peut difficilement être maintenue à moins de 2 % ;
- les travaux de A. BAIKER et J. KIJENSKI (catal. Rev. - Sci. Eng., (27)4, 653-697,1985), qui montrent que la réaction

$$ROH + H_2N-CH_3 \rightarrow RNHCH_3 + H_2O$$

réalisée sur lit fixe de Cu/SiO$_2$ à 220°C conduit à 3% au moins de RN(OH$_3$)$_2$. Le rendement maximum obtenu en RNHCH$_3$ est 75%.

La demanderesse a maintenant découvert qu'il était possible d'accéder aux amines secondaires R-NH-R', en particulier aux méthylalkylamines RNHCH$_3$, par la réaction

(R- ou R'-) OH  +  (R'- ou R-) NH$_2$ → R-NH-R' + H$_2$O ,

selon un procédé dont la sélectivité est très inattendue, et qui se met en oeuvre de façon caractéristique en présence d'hydrogène sur un système catalytique particulier composé d'un catalyseur à base de nickel supporté et d'un carbonate alcalin.

Le système fonctionne dans les deux cas suivants :
- quand la chaîne alkyle à 10/22 atomes de carbone est portée par l'amine RNH$_2$, et qu'on fait réagir le méthanol selon la réaction globale

RNH$_2$  +  CH$_3$OH -> RNHCH$_3$  +  H$_2$O      (1)

- quand la chaîne alkyle à 10/22 atomes de carbone est portée par l'alcool ROH, et qu'on fait réagir la monométhylamine selon la réaction globale

ROH  +  H$_2$NCH$_3$ -> RNHCH$_3$  + H$_2$O      (2)

Le système fonctionne également pour l'obtention d'amines secondaires dissymétriques R-NH-R' où la chaîne courte est un reste à 2/8 atomes de carbone, auquel cas les réactifs à chaîne courte sont respectivement un alcool ou une amine à 2/8 atomes de carbone.

Le mécanisme présumé de l'amination implique que la première étape soit la déshydrogénation de l'alcool. Comme cette réaction est régie par un équilibre thermodynamique très favorable à l'alcool, il est préférable d'avoir en jeu une quantité importante d'alcool, de façon à générer suffisamment d'aldéhyde en équilibre, lequel réagit très rapidement avec l'amine. Or dans le cas de la réaction (1), cela implique de maintenir une quantité importante de méthanol en présence d'amine grasse, plus précisément de maintenir un rapport molaire méthanol/amine compris entre 15 et 30, donc de travailler en réacteur fermé avec une pression autogène de méthanol qui atteint 27 bars à 180°C et 36 bars à 200°C. Une pression d'hydrogène de 10 à 50 bars, préférentiellement d'environ 30 bars, étant au surplus nécessaire pour assurer la sélectivité de la réaction et maintenir l'activité du catalyseur, on arrive à une pression totale importante dans les conditions moyennes de mise en oeuvre de la réaction (1). De surcroît, puisque l'on travaille en réacteur fermé, on ne peut pas chasser l'eau formée au cours de la réaction d'amination, ce qui limite la vitesse de réaction.

Au contraire, dans le cas de la réaction (2) mettant en oeuvre un alcool gras et la monométhylamine, on peut placer dans un autoclave une quantité importante d'alcool gras à une température supérieure à 180°C sous un flux gazeux composé de monométhylamine en excès par rapport à la stoechiométrie (entre 1 et 5 fois la stoechiométrie) et d'hydrogène circulant dans la solution agitée d'alcool gras, en présence du système catalytique Ni/support + K$_2$CO$_3$. Dans ce cas on peut fixer la pression de réaction à la valeur désirée et éliminer en continu l'eau formée dans l'amination qui quitte la phase liquide entraînée par l'hydrogène et l'excès de monométhylamine. Ce mode de réalisation selon la réaction (2) est, pour toutes ces raisons, le mode préféré de l'invention, bien que la mise en oeuvre de la réaction (1) soit chimiquement tout aussi sélective.

La mise en oeuvre du procédé selon l'invention se fait d'une façon générale en suivant l'une ou l'autre des méthodes décrites ci-après.

A) *A partir d'amines grasses et d'alcool léger (réaction 1).*

On introduit dans un autoclave de 3,8 litres (1 US gallon) :
* 500 à 1000 g d'amine grasse à 10/22 atomes de carbone ;
* 500 à 1000 g d'alcool à chaîne courte (1 à 8 atomes de carbone) ;
* 2 % à 10 % en poids de catalyseur (pourcentage exprimé par rapport au poids d'amine grasse) ;
* 0,5 % à 10 % de carbonate de potassium (pourcentage exprimé par rapport au poids d'amine grasse).

On ferme le réacteur et l'on purge plusieurs fois la phase gazeuse sous agitation du liquide à l'aide d'un gaz neutre, avant d'introduire de 10 à 50 bars d'hydrogène. On monte en environ une heure la température de l'autoclave à 180-200°C, sous une agitation de 1800 t/min pendant le temps nécessaire à la disparition quasi totale de l'amine primaire. Après refroidissement et décompression, on filtre la phase liquide et on évapore l'alcool en excès. On lave à l'eau chaude le résidu d'évaporation jusqu'à élimination du $K_2CO_3$.

*B) A partir d'alcool gras et de monométhylamine (réaction 2).*

On introduit dans un autoclave d'un litre :
* 50 à 300 g de monométhylamine ;
* 400 à 600 g d'alcool à chaîne grasse à 10/22 atomes de carbone ;
* 2 % à 10 % poids de catalyseur par rapport à l'alcool gras ;
* 0,5 à 10 % de carbonate de potassium $K_2CO_3$ par rapport à l'alcool gras.

On procède comme précédemment pour charger le réacteur et l'on établit une circulation d'hydrogène dans le réacteur agité à un débit compris entre 20 et 500 l/h, à une pression allant de 10 à 50 bars, préférentiellement à 30 bars. Après un temps défini à l'avance, et préférentiellement après disparition totale de l'alcool gras, on laisse refroidir, on décomprime le réacteur et on filtre le produit. On lave à l'eau chaude le résidu d'évaporation jusqu'à élimination du $K_2CO_3$. On peut, comme il vient d'être indiqué, introduire la monométhylamine en une seule fois ; mais il est encore plus avantageux de procéder à l'introduction de la monométhylamine en continu pendant toute la durée de l'opération. De la même manière, on peut introduire le catalyseur et le carbonate de potassium en une fois, mais on peut aussi l'introduire en deux ou plusieurs fois au cours de l'opération, cette façon de procéder permettant d'atteindre des taux élevés de conversion de l'alcool lourd.

Le système catalytique caractéristique de l'invention est constitué de l'association d'un catalyseur au nickel supporté et d'un carbonate alcalin, plus particulièrement le carbonate de potassium. Une association de ce genre a été décrite dans le brevet français n° 2 351 088 (BASF) mais l'objectif visé était l'obtention exclusive de méthyldialkylamines symétriques $R_2NCH_3$, et la réaction à favoriser était la réaction :

$$ROH + H_2NCH_3 \rightarrow R_2NCH_3 + 2H_2O$$

avec un catalyseur Ni/carbonate de sodium dans un rapport pondéral de 40/1. Les systèmes catalytiques selon la présente invention, qui d'ailleurs diffèrent de ceux du brevet français n° 2 351 088 en ce que le rapport pondéral carbonate alcalin/nickel est compris entre 1/4 et 1/1, développent la réaction avec une sélectivité qui élimine largement les amines secondaires des amines tertiaires produites. Il est tout-à-fait inattendu, et c'est ce qui base la présente invention, que dès qu'on applique une pression suffisante d'hydrogène au système, il développe également une sélectivité, mais dans le sens complètement opposé où ce sont les amines secondaires qui se trouvent favorisées au détriment des amines tertiaires.

Les nickels supportés constituent une classe de catalyseurs bien connue, qui sont préparés par imprégnation ou par précipitation de nitrate de nickel ou de tout autre sel soluble de nickel dont l'anion n'est pas un poison catalytique, sur un support tel que le kieselguhr, l'alumine activée ou de forte surface spécifique, la silice ou la silice alumine, le charbon actif, etc... Les teneurs en nickel peuvent y atteindre des valeurs aussi élevées que 80 %. Certains catalyseurs utiles pour l'invention peuvent être formés de gouttelettes d'un nickel en poudre lié par une amine. Les systèmes carbonate alcalin/Ni + Cu font également partie de l'invention ; on utilise alors de préférence de tels catalyseurs dont le rapport pondéral Ni/Cu est compris entre 100/1 et 10/1.

On utilise les catalyseurs selon la présente invention en quantités comprises entre 1 et 15 % en poids, préférentiellement 2 à 7 %, par rapport au réactif à chaîne grasse mis en oeuvre. On a préféré les produits qui sont cités aux exemples donnés plus loin, et qui sont le catalyseur HARSHAW Ni 1404 T qui comporte 60 % de nickel déposé sur kieselguhr, le Ni 5189D qui contient 18 % en poids de Ni, 2 % en poids de Cu et 7 % en poids d'un support, préférentiellement aluminique, empâté dans de la distéarylamine (on peut aussi empâter dans l'alcool gras si on en utilise dans la réaction), le catalyseur MALLINCKRODT E 230 P, qui contient 60 % en poids de nickel déposé sur alumine, le catalyseur MALLINCKRODT E 480 P, qui contient 65 % en poids de nickel supporté, ou le catalyseur GIRDLER G96, qui contient 23 % en poids de nickel déposé sur silice (6,5 % en poids) et empâté dans de la distéarylamine.

Dans tous les cas, le système catalytique Ni/support + carbonate alcalin en particulier $K_2CO_3$, permet de limiter la teneur en amine tertiaire diméthylée $RN(CH_3)_2$ du produit résultant de l'amination à moins de 2 % et la teneur en amine dialkylée (ultérieurement séparable, si besoin est, par distillation) à moins de 6 %.

Cette sélectivité est une caractéristique de la présente invention.

EXAMPLES

Les exemples suivants feront mieux comprendre l'invention et ses avantages. Dans leur description, on a toujours exprimé les quantités de catalyseur et de carbonate alcalin en poids % rapportées à l'amine lourde ou à l'alcool lourd de départ (à 10/22 atomes de carbone).

EXEMPLES 1 et 2 : production de N-méthyl-N-dodécylamine à partir de dodécylamine et de méthanol.

On suit le mode opératoire donné plus haut, avec pour conditions particulières une pression d'hydrogène de 40 bars et une agitation à 1800 t/min pendant 6 heures à 180°C. Pour l'essai n°1 selon l'invention, les réactifs introduits dans l'autoclave sont les suivants :
*   422 g de dodécylamine (2,3 moles),
*   1460 g de méthanol (45 moles),
*   38,6 g de catalyseur HARSHAW Ni 1404 T,
*   57,1 g de $K_2CO_3$.

Un contre-exemple, (essai n°2) est réalisé dans les mêmes conditions, mais sans introduction du carbonate de potassium. Les résultats obtenus sont reproduits sur le tableau I ci-après :

## TABLEAU I

| | | PRODUITS DE REACTION (poids exprimé en pourcentage) | | | | |
|---|---|---|---|---|---|---|
| *ESSAI* | $K_2CO_3$ | $RNH_2$ | $RNHCH_3$ | $RN(CH_3)_2$ | $R_2NH$ | $ROH$ |
| 1 | oui | – | 85,9 | 1,3 | 6,0 | 6,8 |
| 2 | non | 15,0 | 17,2 | 44,9 | 13,8 | 9,1 |

Ces résultats montrent clairement que la présence de $K_2CO_3$ dans le milieu permet la formation sélective de N-méthyl-dodécylamine et qu'elle augmente l'activité du catalyseur.

EXEMPLES 3 à 5 : production de N-méthyl-dodécylamine à partir de dodécylamine et de méthanol.

Sur le tableau II, on a rapproché les résultats de trois essais effectués en faisant varier le rapport molaire méthanol/amine (ci-après M/A) entre 5 et 15, sous 40 bars d'hydrogène à 180°C pendant 6 heures, en présence de 9,1 % de catalyseur HARSHAW Ni 1404 T et de 13,5 % de $K_2CO_3$, de ceux de l'exemple 1, où le rapport M/A était de 20.

**TABLEAU II**

| ESSAI | M/A | PRODUITS DE REACTION (poids exprimé en pourcentage) | | | | |
|-------|-----|------|--------|-----------|--------|------|
| | | $RNH_2$ | $RNHCH_3$ | $RN(CH_3)_2$ | $R_2NH$ | $ROH$ |
| 3 | 5 | 46,7 | 48,2 | 0,8 | 2,4 | 2,5 |
| 4 | 10 | 17,1 | 75,4 | 0,3 | 3,8 | 3,8 |
| 5 | 15 | 05,6 | 82,4 | 1,7 | 5,5 | 4,8 |
| 1 | 20 | – | 85,9 | 1,3 | 6,0 | 6,8 |

Ces exemples montrent l'influence du rapport molaire M/A du méthanol à la monométhylamine : son augmentation permet d'améliorer l'activité en méthylation sans dépasser 2 % en diméthyldodécylamine.

EXEMPLES 6 à 10

On a reproduit sur le tableau III des résultats de préparation de N-éthyl-dodécylamine (essai n°6), de N-isopropyl-dodécylamine (essai n°7), de N-méthyl-alkyl(coprah)amine (essai n°8), de N-méthyl-alkyl(suif)-amine (essai n°9) et de N-méthyl-alkyl(C20/C22)amine (essai n°10), à partir des amines grasses et des alcools légers correspondants. Sur ce tableau, on a attribué le symbole R aux chaînes lourdes (C12-coprah, suif, C20/22) et le symbole R' aux chaînes courtes (C1/3).

On a effectué des essais à partir des matières premières indiquées dans le tableau III en présence de catalyseur HARSHAW Ni 1404 T et de $K_2CO_3$, à 180°C sous 50 bars d'hydrogène. Le rapport molaire alcool/amine a été fixé ici à 10. On lit sur ce tableau que quels que soient les réactifs de départ, la teneur en amine $RN(R')_2$ reste inférieure à 2 % en poids.

6

**TABLEAU III**

| Essai | Réactifs | Catalyseur % poids | $K_2CO_3$ % poids | Temps de réaction (h) | PRODUITS DE REACTION (en poids) | | | |
|---|---|---|---|---|---|---|---|---|
| | | | | | RNH2 | RNHR' | RN(R')$_2$ | $R_2NH$ + ROH |
| 6 | Dodécylamine + éthanol | 9,2 | 13,5 | 19,0 | 1,9 | 66,0 | 1,9 | 30,2 |
| 7 | Dodécylamine + isopropanol | 9,2 | 13,5 | 19,0 | 2,3 | 90,0 | 0,5 | 7,2 |
| 8 | Amine de coprah + méthanol | 7,7 | 11,3 | 06,5 | 24,0 | 64,0 | 1,2 | 10,8 |
| 9 | Amine de suif + méthanol | 5,7 | 6,3 | 26,0 | 2,3 | 84,0 | 1,5 | 12,2 |
| 10 | Amine C20/22 + méthanol | 5,7 | 8,3 | 15,0 | 9,7 | 69,0 | 1,7 | 19,6 |

On a reproduit sur le tableau IV des résultats de préparation de N-méthyldodécylamine à partir de dodécylamine et de méthanol, selon les conditions utilisées dans l'exemple 1, mais en utilisant des carbonates de lithium, de sodium ou de potassium dans des proportions variables.

Ces exemples montrent que la présence de carbonates alcalins est favorable à la formation sélective de N-méthyldodécylamine et que cet effet est le plus marqué avec $K_2CO_3$.

## TABLEAU IV

| | ADDITIF | | PRODUITS DE REACTION (poids exprimé en %) | | | |
|---|---|---|---|---|---|---|
| Essai | Type | % poids /amines | $RN(CH_3)_2$ | $RNHCH_3$ | $R_2NH$ | Non dosés |
| 11 | $Li_2CO_3$ | 1 | 14 | 31 | 42 | 13 |
| 12 | $Li_2CO_3$ | 10 | 10 | 28 | 21 | 41 |
| 13 | $Na_2CO_3$ | 5 | 21 | 55 | 6 | 18 |
| 14 | $Na_2CO_3$ | 10 | 55 | 39 | 2 | 4 |
| 15 | $Na_2CO_3$ | 15 | 12 | 60 | 17 | 11 |
| 16 | $K_2CO_3$ | 5 | 3 | 32 | 61 | 4 |
| 17 | $K_2CO_3$ | 10 | 2 | 80 | 0 | 18 |
| 18 | $K_2CO_3$ | 15 | 2 | 78 | 2 | 18 |

EXEMPLES 19 ET 20

On a reproduit sur le tableau V des résultats de préparations de N-méthyl-alkyl(C12/14)amines à partir de l'alcool gras et de monométhylamine, selon le mode opératoire général décrit plus haut, et dans lesquelles on a chargé 466 g d'alcools en coupe C12/C14 (75 % de dodécanol/25 % de tétradécanol), 5 % par rapport à l'alcool de catalyseur au nickel supporté, 5 % par rapport à l'alcool de carbonate de potassium. La pression est de 10 bars, le débit d'hydrogène de 100 l/h, le débit de monométhylamine de 40 g/heure, et la durée totale des opérations de 7 heures.

Ces exemples montrent clairement l'effet spécifique de l'additif $K_2CO_3$ pour la sélectivité de la synthèse des N-méthyl-alkylamines.

## TABLEAU V

| | | T° | Conversion | PRODUITS DE REACTION (% en poids) | | | | |
|---|---|---|---|---|---|---|---|---|
| Essai | Catalyseur | C° | ROH (% poids) | $RNH_2$ | $RNHCH_3$ | $RN(CH_3)_2$ | Dialkyl amines | Trialkyl amines |
| 19 | Ni 1404 T (seul) | 200 | 100 | 1,4 | 3 | 0 | 68,3 | 27,3 |
| 20 | Ni 1404 T + $K_2CO_3$ | 200 | 75,4 | 6,8 | 72,7 | 2,5 | 17,1 | 0,9 |

8

EXEMPLES 21 A 25

On a reproduit sur le tableau VI des résultats de préparation de N-méthyl-dodécylamine à partir de dodécanol et de monométhylamine, selon le mode opératoire général décrit plus haut, et dans lequel on a chargé 433 g de dodécanol, 5 % par rapport à l'alcool de catalyseur au nickel supporté, 2,5% par rapport à l'alcool de carbonate de potassium. La température a été choisie à 200°C, le débit d'hydrogène était de 100 l/h, le débit de monométhylamine de 26 g/h et la durée des opérations de 7 heures. La pression a été de 10 bars.

## TABLEAU VI

| | | Conversion | PRODUITS DE REACTION (% en poids) | | | | |
|---|---|---|---|---|---|---|---|
| Essai | Catalyseur | ROH (% poids) | $RNH_2$ | $RNHCH_3$ | $RN(CH_3)_2$ | Dialkyl amines | Trialkyl amines |
| 21 | Ni 1404 T | 75 | 6,8 | 72,7 | 2,5 | 17,1 | 0,9 |
| 22 | Ni 8849 D + $K_2CO_3$ | 68 | 2,3 | 85,1 | 0,2 | 9,9 | 2,5 |
| 23 | E 230 P + $K_2CO_3$ | 84 | 1,2 | 75,4 | 1,1 | 22,1 | 0,2 |
| 24 | G 96 + $K_2CO_3$ | 69 | 1,0 | 93,9 | 0,1 | 5,0 | 0 |
| 25 | E 480 P + $K_2CO_3$ | 85 | 0,4 | 75,8 | 2,4 | 21,4 | 0 |

EXEMPLES 26 A 28

On a reproduit sur le tableau VII des résultats de préparation de N-méthyl-dodécylamine à partir de dodécanol et de monométhylamine, selon le mode opératoire et les conditions des exemples 21 à 25, et dans lesquels on a changé uniquement la pression totale d'hydrogène entre 3 et 30 bars, en utilisant cette fois le catalyseur Ni 5189 D.

## TABLEAU VII

| Pression H2 (Bars) | Conversion ROH (% en poids) | SELECTIVITE (poids exprimé en %) | | | |
|---|---|---|---|---|---|
| | | $RNH_2$ | $RNHCH_3$ | $RN(CH_3)_2$ | Dialkyl Amines |
| 3 | 61 | 2,6 | 73,1 | 1,7 | 22,3 |
| 10 | 59 | 3,7 | 89,3 | 0,5 | 4,6 |
| 30 | 50 | 3,6 | 90,4 | 1,0 | 2,4 |

L'exemple montre l'influence très favorable de l'hydrogène qui évite la formation massive de dialkylamines.

EXEMPLES 29 A 31

On a reproduit sur le tableau VIII des résultats de préparation de N-méthyl-dodécylamine à partir de dodécanol et de monométhylamine, selon le mode opératoire et les conditions des exemples 21 à 25, la pression ayant été de 10 bars, la température de 200°C, le débit de monométhylamine de 26 g/h. A la différence des exemples 21 à 25, on a ajouté le catalyseur en deux fois au cours de l'opération, ainsi qu'indiqué sur le tableau VIII, la durée totale de la réaction dépassant 7 heures. On obtient ainsi un produit répondant aux spécifications générales de teneur en amine diméthylée et en amine primaire avec un taux de conversion supérieur à 98 %.

**TABLEAU VIII**

| | Catalyseur | | | | | | Sélectivite (% poids) | | | |
|---|---|---|---|---|---|---|---|---|---|---|
| Essai | Type | % poids | $K_2CO_3$ %poids | Temps de réaction (h) | ROH converti (% pds) | $RNH_2$ | $RNH$ \| $CH_3$ | $RN \stackrel{CH_3}{_{CH_3}}$ | Dialkyl amines | Trialkyl amines |
| 26 | Ni 8849D | 5 | 0,6 | 7 | 61 | 3,1 | 87,4 | 0 | 9,5 | 0 |
| | | +5 | +0,6 | +7 | 98 | 2,5 | 78,1 | 1,5 | 16,3 | 1,6 |
| 27 | E 480 P | 2,5 | 1,25 | 7 | 71 | 0,4 | 88,1 | 0,1 | 11,4 | 0 |
| | | +2,5 | +1,25 | +7 | 99 | 0,4 | 76,1 | 2,5 | 21,0 | 0 |
| 28 | E 230 P | 2,5 | 1,25 | 7 | 68 | 0,1 | 82,3 | 0,2 | 17,4 | 0 |
| | | +2,5 | +1,25 | +7 | 99 | 0,4 | 83,5 | 1,7 | 14,4 | 0 |

**Revendications**

1.  Procédé pour la préparation d'amines secondaires aliphatiques dissymétriques de formule générale R-NH-R'

    où R est une chaîne aliphatique comportant de 10 à 22 atomes de carbone, et R' une chaîne aliphatique courte comportant de 1 à 8 atomes de carbone, par réaction d'amination catalysée par un catalyseur au nickel supporté en présence d'un carbonate alcalin entre un alcool et une monoalkylamine, caractérisé en ce que la réaction est effectuée sous une pression d'hydrogène comprise entre 10 et 50 bars.

2.  Procédé pour la préparation d'amines secondaires aliphatiques dissymétriques selon la revendication 1, caractérisé en ce que la pression d'hydrogène sous laquelle est effectuée la réaction d'amination est d'environ 30 bars.

3.  Procédé selon les revendications 1 ou 2, caractérisé en ce que la réaction d'amination est effectuée entre un alcool gras comportant de 10 à 22 atomes de carbone, et une monoalkylamine comportant de 1 à 8 atomes de carbone.

4.  Procédé selon les revendications 1 ou 2, caractérisé en ce que la réaction d'amination est effectuée entre une monoamine grasse comportant de 10 à 22 atomes de carbone, et un alcanol comportant de 1 à 8 atomes de carbone.

**EP 0 526 318 B1**

5. Procédé selon la revendication 4, caractérisé en ce que l'alcanol est le méthanol.

6. Procédé selon la revendication 5, caractérisé en ce que le méthanol est introduit en quantité telle que le rapport molaire entre le méthanol et l'amine soit compris entre 15 et 30.

7. Procédé selon la revendication 3, caractérisé en ce que la monoalkylamine est la monométhylamine.

8. Procédé selon la revendication 7, caractérisé en ce que le rapport molaire entre la monométhylamine et l'alcool gras est compris entre 1 et 5.

9. Procédé selon l'une quelconque des revendications 1 à 4, caractérisé en ce que le carbonate alcalin est le carbonate de potassium.

10. Procédé selon l'une quelconque des revendications 1 à 4, caractérisé en ce que le rapport pondéral entre le carbonate alcalin et le catalyseur au nickel est compris entre 1/4 et 1/1.

11. Procédé selon l'une quelconque des revendications 1 à 4, caractérisé en ce que le catalyseur au nickel supporté est un catalyseur mixte nickel/cuivre.

12. Procédé selon l'une quelconque des revendications 1 à 4, caractérisé en ce que la quantité de catalyseur est comprise entre 1 et 15% en poids par rapport au réactif à chaîne grasse mis en oeuvre.

**Claims**

1. Process for the preparation of unsymmetrical aliphatic secondary amines of general formula

   R-NH-R'

   where R is an aliphatic chain containing from 10 to 22 carbon atoms and R' is a short aliphatic chain containing from 1 to 8 carbon atoms, by an amination reaction, catalysed by a supported nickel catalyst in the presence of an alkali metal carbonate, between an alcohol and a monoalkylamine, characterized in that the reaction is carried out at a hydrogen pressure between 10 and 50 bar.

2. Process for the preparation of unsymmetrical aliphatic secondary amines according to Claim 1, characterized in that the hydrogen pressure at which the amination reaction is carried out is approximately 30 bar.

3. Process according to Claims 1 or 2, characterized in that the amination reaction is carried out between a fatty alcohol containing from 10 to 22 carbon atoms and a monoalkylamine containing from 1 to 8 carbon atoms.

4. Process according to Claims 1 or 2, characterized in that the amination reaction is carried out between a fatty monoamine containing from 10 to 22 carbon atoms and an alkanol containing from 1 to 8 carbon atoms.

5. Process according to Claim 4, characterized in that the alkanol is methanol.

6. Process according to Claim 5, characterized in that the methanol is introduced in an amount such that the molar ratio between the methanol and the amine is between 15 and 30.

7. Process according to Claim 3, characterized in that the monoalkylamine is monomethylamine.

8. Process according to Claim 7, characterized in that the molar ratio between the monomethylamine and the fatty alcohol is between 1 and 5.

9. Process according to any one of Claims 1 to 4, characterized in that the alkali metal carbonate is potassium carbonate.

11

10. Process according to any one of Claims 1 to 4, characterized in that the weight ratio between the alkali metal carbonate and the nickel catalyst is between 1/4 and 1/1.

11. Process according to any one of Claims 1 to 4, characterized in that the supported nickel catalyst is a nickel/copper mixed catalyst.

12. Process according to any one of Claims 1 to 4, characterized in that the amount of catalyst is between 1 and 15% by weight relative to the fatty-chain-containing reactant used.

**Patentansprüche**

1. Verfahren zur Herstellung von sekundären aliphatischen unsymmetrischen Aminen der allgemeinen Formel

   R-NH-R'

   in der R eine aliphatische Kette mit 10 bis 22 Kohlenstoffatomen und R' eine kurze aliphatische Kette mit 1 bis 8 Kohlenstoffatomen ist, bestehend aus der mit einem Nickelkatalysator auf einem Tragermaterial in Gegenwart eines Alkalicarbonats katalysierte Aminierungsreaktion zwischen einem Alkohol und einem Monoalkylamin, dadurch gekennzeichnet, daß die Reaktion bei einem Wasserstoffdruck zwischen 10 und 50 bar durchgeführt wird.

2. Verfahren zur Herstellung von sekundären aliphatischen unsymmetrischen Aminen nach Anspruch 1, dadurch gekennzeichnet, daß der Wasserstoffdruck, bei den, die Aminierungsreaktion durchgeführt wird, ungefähr 30 bar beträgt.

3. Verfahren nach Anspruch 1 oder 2, dadurch gekennzeichnet, daß die Aminierungsreaktion mit einem Fettalkohol mit 10 bis 20 Kohlenstoffatomen und einem Monoalkylamin mit 1 bis 8 Kohlenstoffatomen durchgeführt wird.

4. Verfahren nach Anspruch 1 oder 2, dadurch gekennzeichnet, daß die Aminierungsreaktion mit einen, Fettmonoamin mit 10 bis 22 Kohlenstoffatomen und einen, Alkanol mit 1 bis 8 Kohlenstoffatomen durchgeführt wird.

5. Verfahren nach Anspruch 4, dadurch gekennzeichnet, daß das Alkanol Methanol ist.

6. Verfahren nach Anspruch 5, dadurch gekennzeichnet, daß das Methanol in einer solchen Menge zugeführt wird, daß das molare Verhältnis zwischen dem Methanol und dem Amin zwischen 15 und 30 liegt.

7. Verfahren nach Anspruch 3, dadurch gekennzeichnet, daß das Monoalkylamin Monomethylamin ist.

8. Verfahren nach Anspruch 7, dadurch gekennzeichnet, daß das Molverhältnis zwischen dem Monomethylamin und dem Fettalkohol zwischen 1 und 5 liegt.

9. Verfahren nach einem der Ansprüche 1 bis 4, dadurch gekennzeichnet, daß das Alkalicarbonat Kaliumcarbonat ist.

10. Verfahren nach einem der Ansprüche 1 bis 4, dadurch gekennzeichnet, daß das Gewichtsverhältnis zwischen dem Alkalicarbonat und dem Nickelkatalysator zwischen 1/4 und 1/1 liegt.

11. Verfahren nach einem der Ansprüche 1 bis 4, dadurch gekennzeichnet, daß der Nickelkatalysator auf einem Trägermaterial ein gemischter Nickel/Kupferkatalysator ist.

12. Verfahren nach einem der Ansprüche 1 bis 4, dadurch gekennzeichnet, daß die Katalysatormenge zwischen 1 und 15 Gew.-% in bezug auf das eingesetzte Reagenz mit der Fettsäurekette liegt.